(19) Europäisches Patentamt
European Patent Office
Office européen des brevets

(11) EP 1 200 400 B1

(12) EUROPÄISCHE PATENTSCHRIFT

(45) Veröffentlichungstag und Bekanntmachung des Hinweises auf die Patenterteilung:
**29.09.2004 Patentblatt 2004/40**

(21) Anmeldenummer: **00945950.4**

(22) Anmeldetag: **25.07.2000**

(51) Int Cl.[7]: **C07D 207/02**, A61K 31/4015, A61P 25/04, A61P 29/00

(86) Internationale Anmeldenummer:
**PCT/EP2000/007101**

(87) Internationale Veröffentlichungsnummer:
**WO 2001/010831 (15.02.2001 Gazette 2001/07)**

(54) **SUBSTITUIERTE PYRROLIDIN-2,3,4-TRION-DERIVATE WIRKSAM ALS NMDA-REZEPTOR-ANTAGONISTEN**

SUBSTITUTED PYRROLIDIN-2,3,4-TRION DERIVATIVES USEFUL AS NMDA-RECEPTOR ANTAGONISTS

DERIVES SUBSTITUES DE PYRROLODINE-2,3,4-TRION AGISSANT EN TANT QU'ANTAGONISTES DU RECEPTEUR NMDA

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **06.08.1999 DE 19936521**

(43) Veröffentlichungstag der Anmeldung:
**02.05.2002 Patentblatt 2002/18**

(73) Patentinhaber: **Grünenthal GmbH**
**52078 Aachen (DE)**

(72) Erfinder:
- **PRZEWOSNY, Michael**
  **D-52062 Aachen (DE)**
- **STACHEL, Hans-Dietrich**
  **D-82061 Neuried (DE)**
- **POSCHENRIEDER, Hermann**
  **D-82166 Gräfelfing (DE)**

(56) Entgegenhaltungen:
**EP-A- 0 894 497** **WO-A-96/08485**

- **POSCHENRIEDER, H.: "5-Aryliden-pyrrolidine-2,3,4-trione 3 Oximes as NMDA Receptor Antagonist" ARCH. PHARM., Bd. 332, Nr. 9, 1999, Seiten 309-316, XP002146311 Weinheim, Ger.**
- **DATABASE CHEMABS [Online] CHEMICAL ABSTRACTS SERVICE, COLUMBUS, OHIO, US; POSCHENRIEDER, HERMANN ET AL: "5-Arylidene-3-aryl-pyrrolidine-2,4-diones with affinity to the N-methyl-D-aspartate (glycine site) receptor. Part 1" retrieved from STN Database accession no. 130:139224 XP002146885 & ARCH. PHARM. (WEINHEIM, GER.) (1998), 331(12), 389-394 ,**
- **ROWLEY M ET AL: "ROUTES TO 4-SUBSTITUTED ANALOGUES OF THE GLYCINE/NMDA ANTAGONIST HA-966. ENANTIOSELECTIVE SYNTHESIS OF (3R,4R) 3-AMINO-1-HYDROXY-4-METHYL-2-PYRROLIDINONE" TETRAHEDRON,NL,ELSEVIER SCIENCE PUBLISHERS, AMSTERDAM, Bd. 48, Nr. 17, 1992, Seiten 3557-3570, XP000604668 ISSN: 0040-4020**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 200 400 B1

## Beschreibung

Die Erfindung betrifft substituierte Pyrrolidin-2,3,4-trion-Derivate, Verfahren zu deren Herstellung, Arzneimittel enthaltend diese Verbindungen, sowie die Verwendung dieser Verbindungen zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie z.B. Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation, Sucht, Abhängigkeit und Toleranzentwicklung limitiert. Sie können daher nur unter besonderen Vorsichtsmaßnahmen wie z.B. speziellen Verordnungsvorschriften über einen längeren Zeitraum oder in höheren Dosierungen gegeben werden (Goodman, Gilman, The Pharmacological Basis of Therapeutics, Pergamon Press, New York 1990). Außerdem zeigen sie bei einigen Schmerzzuständen, insbesondere bei neuropathischen und inzidentiellen Schmerzen, eine geringere Wirksamkeit.

Opioide entfalten ihre analgetische Wirkung durch Bindung an membranständige Rezeptoren, die zur Familie der sogenannten G-Protein-gekoppelten Rezeptoren gehören. Daneben gibt es weitere Rezeptoren und Ionenkanäle, die wesentlich an dem System der Schmerzentstehung und der Schmerzweiterleitung beteiligt sind, wie z.B. der N-Methyl-D-Aspartat-(NMDA)-Ionenkanal, über den ein wesentlicher Teil der Kommunikation von Synapsen abläuft und durch den der Calcium-Ionenaustausch zwischen einer neuronalen Zelle und ihrer Umgebung gesteuert wird.

Kenntnisse über die physiologische Bedeutung von Ionenkanal-selektiven Substanzen sind durch die Entwicklung der patch-clamp-Technik gewonnen worden, mit der sich die Wirkung von NMDA-Antagonisten auf den Calciumhaushalt im Zellinneren nachweisen läßt.

Aus der Literaturveröffentlichung von Stachel et al., Arch. Pharm. Pharm. Med. Chem., 1998, 331, 389-394 sind 5-Aryliden-3-aryl-pyrrolidin-2,4-dione und deren Affinität zum NMDA-Rezeptor bekannt.

Der WO 96/08485 sind substituierte Chinoxalindion-Verbindungen mit NMDAantagonistischer Wirkung zu entnehmen.

Die EP 0 894 497 offenbart die Verwendung substituierter Imidazolin-2,4-dion-Verbindungen in Arzneimitteln zur Bekämpfung von Schmerzen.

Aus der Veröffentlichung von Rowley et al. Tetrahedron, 48, Nr. 17, 1992, Seiten 3557-3570 sind 4-substituierte Analoga des NMDA-Antagonisten 3-Amino-1-hydroxy-2-pyrrolidinon sowie Verfahren zu deren Herstellung bekannt.

Der nach dem Prioritätstag dieser Anmeldung veröffentlichten Literatur von Stachel et al., Arch. Pharm. Pharm. Med. Chem., 1999, 332, 309-316 sind 5-Arylidenpyrrolidin-2,3,4-trion-3-oxime und deren Eignung zur Verwendung als NMDA-Rezeptor-Antagonisten zu entnehmen.

Eine der Erfindung zugrundeliegende Aufgabe bestand in dem zur Verfügung stellen von neuen Verbindungen, die sich zur Schmerztherapie oder zur Anxiolyse eignen. Darüber hinaus sollten diese Verbindungen möglichst wenig Nebenwirkungen der Opioid-Analgetika wie z.B. Übelkeit, Erbrechen, Abhängigkeit, Atemdepression oder Obstipation aufweisen. Weitere Aufgaben bestanden darin, neue Wirkstoffe zur Behandlung von inflammatorischen und/oder allergischen Reaktionen, Depressionen, Drogen- und/oder Alkoholmißbrauch, Gastritis, Diarrhoe, Haminkontinenz, cardiovaskulären Erkrankungen, Atemwegserkrankungen, Husten, seelischen Erkrankungen, Epilepsie, Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Hirnödemen, Hypoxie, Anoxie, AIDS-Demens, Encephalomyelitis, Tourette-Syndrom oder perinataler Asphyxie zur Verfügung zu stellen.

Es wurde nun gefunden, daß substituierte Pyrrolidin-2,3,4-trion-Derivate der nachstehenden allgemeinen Formel I als NMDA-Antagonisten selektiv an der Glycin-Bindungsstelle angreifen und sich zur Behandlung von inflammatorischen und/oder allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen, Epilepsie, Schizophrenie, Morbus Alzheimer, Morbus Huntington, Morbus Parkinson, cerebralen ischämien, cerebralen Infarkten, Psychosen bedingt durch erhöhten Aminosäurespiegel, Schlaganfällen, Hirnödemen, Hypoxie, Anoxie, AIDS-Demens, Encephalomyelitis, Tourette-Syndrom oder perinataler Asphyxie eignen und die außerdem eine ausgeprägte analgetische oder anxiolytische Wirkung aufweisen.

Gegenstand der vorliegenden Erfindung sind daher Verbindungen der allgemeinen Formel I,

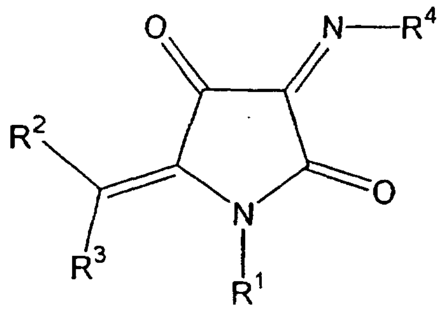

I

worin

der Rest $R^1$ für H, $OR^8$, $COR^5$, $CSR^5$, $NR^6R^7$, $COOR^5$, $CONR^6R^7$, $CSNR^6R7$, einen $C_{1-10}$-Alkyl-, vorzugsweise einen $C_{1-6}$-Alkyl-, einen Aryl-, einen Heteroaryloder für einen über eine $C_{1-6}$-Alkylen-Gruppe, vorzugsweise für einen über eine $C_{1-3}$-Alkylen-Gruppe gebundenen Aryl-Rest steht,

die Reste $R^2$, $R^3$, gleich oder verschieden, für H, F, Cl, Br, $CF_3$, $OR^8$, $SR^8$, einen $C_{1-10}$-Alkyl-, vorzugsweise für einen $C_{1-6}$-Alkyl-, einen Aryl-, einen Heteroaryl-Rest oder für einen über eine $C_{1-6}$-Alkylen-Gruppe, vorzugsweise für einen über eine $C_{1-3}$-Alkylen-Gruppe gebundenen Aryl-Rest stehen,

der Rest $R^4$ für H, OH, $OR^8$, $SR^8$, $COR^5$, $COOR^5$, $COCOR^5$, $CONR^6R^7$, $CSNR^6R^7$, vorzugsweise für OH oder $OR^8$, einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest stehen,

der Rest $R^5$ für H, einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest steht,

die Reste $R^6$, $R^7$, gleich oder verschieden, für H, $OR^8$, $COR^5$, $COOR^5$, einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest stehen,

der Rest $R^8$ für einen $C_{1-10}$-Alkyl-, vorzugsweise einen $C_{1-6}$-Alkyl-, einen Aryl-, einen Heteroaryl- oder für einen über eine $C_{1-6}$-Alkylen-Gruppe gebundenen, vorzugsweise für einen über eine $C_{1-3}$-Alkylen-Gruppe gebundenen Aryl-Rest steht,

in Form ihrer Racemate, Enantiomere, Diastereomere oder einer entsprechenden Base oder eines entsprechenden physiologisch verträglichen Salzes.

Unter Alkyl-Resten werden auch verzweigte, unverzweigte oder cyclische unsubstituierte oder mindestens einfach, vorzugsweise mit F, Cl, Br, CN, $NO_2$, CHO, $SO_2C_{1-6}$-Alkyl, $SO_2CF_3$, $OR^5$, $NR^6R^7$, $COR^5$, $COOR^5$, $COCOR^5$, $CONR^6R^7$ oder $CSNR^6R^7$ substituierte Kohlenwasserstoffe verstanden, wobei die Reste $R^5$ bis $R^7$ die Bedeutung gemäß der allgemeinen Formel I haben. Enthalten diese Alkyl-Reste mehr als einen Substituenten, so können diese gleich oder verschieden sein. Vorzugsweise sind die Alkylreste Methyl, Ethyl, Propyl, Isopropyl, n-Butyl, sek-Butyl, tert-Butyl, Neopentyl, n-Hexyl, Cyclopropyl, Cyclobutyl, Cyclopentyl oder Cyclohexyl.

Unter einem Aryl-Rest werden auch unsubstituierte oder mindestens einfach mit OH, F, Cl, Br, $CF_3$, CN, $NO_2$, CHO, $SO_2C_{1-6}$-Alkyl, $SO_2CF_3$, $OR^5$, $NR^6R^7$, $COR^5$, $COOR^5$, $COCOR^5$, $CONR^6R^7$, $CSNR^6N^7$, einem $C_{1-6}$-Alkyl-Rest, einem $C_{1-6}$-Alkoxy-Rest, einem $C_{2-6}$-Alkylen-Rest, einem Heterocyclyl-Rest und/oder einem Phenyl-Rest substituierte Phenyl-Reste verstanden, wobei die Reste $R^5$ bis $R^7$ die Bedeutung gemäß der allgemeinen Formel I haben. Der Ausdruck kann auch einen ggf. substituierten Naphthyl-Rest bedeuten. Die Phenyl-Reste können auch mit weiteren Ringen kondensiert sein.

Unter einem Heteroaryl-Rest werden auch 5- oder 6-gliedrige ungesättigte, gegebenenfalls mit einem ankondensierten Aryl-Rest versehene, heterocyclische Verbindungen verstanden, die wenigstens ein Heteroatom, vorzugsweise Stickstoff und/oder Sauerstoff und/oder Schwefel enthalten.

Vorzugsweise ist der Heteroaryl-Rest Furan, Thiophen, Pyrrol, Pyridin, Pyrimidin, Chinolin, Isochinolin, Phthalazin oder Chinazolin.

Besonders bevorzugt sind folgende substituierte Pyrrolidin-2,3,4-trion-3-oxim-Derivate:

5-(Methoxyphenylmethylen)-pyrrolidin-2,3,4-trion-3-oxim und

5-(Bromphenylmethylen)-pyrrolidin-2,3,4-trion-3-oxim.

Ein weiterer Gegenstand der vorliegenden Erfindung sind Verfahren zur Herstellung von substituierten Pyrrolidin-2,3,4-trion-Derivaten der allgemeinen Formel I, in denen man Tetramsäuren der allgemeinen Formel II,

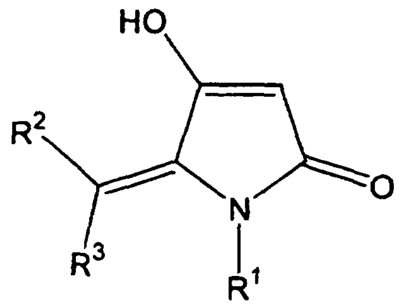

II

worin die Reste $R^1$ bis $R^3$ die Bedeutung gemäß der allgemeinen Formel I haben, in eisgekühlter Lösung, vorzugsweise in eisgekühlter Lösung von Eisessig mit einer wäßrigen Lösung von Natriumnitrit zu Verbindungen der allgemeinen Formel I, worin der Rest $R^4$ für OH steht und die Reste $R^1$ bis $R^3$ die Bedeutung gemäß der allgemeinen Formel I haben, umsetzt, und diese vorzugsweise durch Umkristallisation, bevorzugt aus Ethanol reinigt und isoliert.

Die Synthese der Ausgangsverbindungen, der Tetramsäuren der allgemeinen Formel II, kann nach H. Poschenrieder et al. (Arch. Pharm. Pharm. Med. Chem. 1998, Vol. 331, Seiten 389-394) und Stachel et al. (J. Heterocycl. Chem. 1980, Vol. 17, Seiten 1195-1199 und Liebigs Ann. Chem. 1985, Seiten 1692-1696) sowie den darin zitierten Literaturstellen durchgeführt werden. Sie werden hiermit als Referenz eingeführt und gelten somit als Teil der Offenbarung.

Die Verbindungen der allgemeinen Formel I, worin der Rest $R^4$ für OH steht und die Reste $R^1$ bis $R^3$ die Bedeutung gemäß der allgemeinen Formel I haben, werden vorzugsweise unter Schutzgasatmosphäre in absoluten Lösungsmitteln, vorzugsweise in offenkettigen und/oder cyclischen Ethern bei niedrigen Temperaturen in Gegenwart von starken Basen, vorzugsweise Alkalihydroxiden und/oder Erdalkalihydroxiden und/oder metallorganischen Basen mit $C_{1-10}$-Alkylhalogeniden, vorzugsweise mit $C_{1-6}$-Alkylhalogeniden; mit Arylhalogeniden, Heteroarylhalogeniden oder mit Aryl-$C_{1-6}$-Alkylhalogeniden, vorzugsweise mit Aryl-$C_{1-3}$-Alkylhalogeniden zu Verbindungen der allgemeinen Formel I umgesetzt, worin der Rest $R^4$ für $OR^8$ steht und die Reste $R^1$ bis $R^3$ sowie $R^8$ die Bedeutung gemäß der allgemeinen Formel I haben.

Die Verbindungen der allgemeinen Formel I, worin der Rest $R^4$ für $OR^8$ steht und die Reste $R^1$ bis $R^3$ sowie $R^8$ die Bedeutung gemäß der allgemeinen Formel I haben, können noch weiter derivatisiert werden, in dem sie vorzugsweise unter Schutzgasatmosphäre in einem absoluten Lösungsmittel, vorzugsweise in offenkettigen und/oder cyclischen Ethern, mit Säurechloriden der allgemeinen Formel $R^5$-(C=O)-Cl und/oder Säurebromiden der allgemeinen Formel $R^5$-(C=O)-Br oder Chlorameisensäureestern der allgemeinen Formel Cl-(C=O)-O-$R^5$ bzw. Fluorameisensäureestern der allgemeinen Formel F-(C=O)-O-$R^5$ oder mit offenkettigen Carbonaten der allgemeinen Formel $R^5$-O-(C=O)-O-$R^5$ bzw. mit entsprechend substituierten cyclischen Carbonaten, vorzugsweise mit entsprechend substituierten cyclischen Carbonaten, die 5 oder 6 Atome im Ring aufweisen, worin jeweils der Rest $R^5$ die Bedeutung gemäß der allgemeinen Formel I hat, zu Verbindungen der allgemeinen Formel I, worin der Rest $R^4$ für $COR^5$ und $COOR^5$ steht und die Reste $R^1$ bis $R^3$ sowie der Rest $R^5$ die Bedeutung gemäß der allgemeinen Formel I haben, umgesetzt, und nach den üblichen Verfahren gereinigt und isoliert werden.

Die Verbindungen der allgemeinen Formel I, worin der Rest $R^4$ für OH steht und die Reste $R^1$ bis $R^3$ die Bedeutung gemäß der allgemeinen Formel I haben, können auch mit aliphatischen, aromatischen bzw. heteroaromatischen Isocyanaten oder Isothiocyanaten bei niedrigen Temperaturen in aprotischen, polaren Lösungsmitteln zu Verbindungen der allgemeinen Formel I, worin der Rest $R^4$ für $CONR^6R^7$ oder $CSNR^6R^7$ steht, der Rest $R^6$ oder $R^7$ H bedeutet und die Reste $R^1$ bis $R^3$ sowie $R^6$ oder $R^7$ die Bedeutung gemäß der allgemeinen Formel I haben, umgesetzt werden, und nach den üblichen Verfahren gereinigt und isoliert werden.

Die Herstellung der Verbindungen der allgemeinen Formel I, in denen der Rest $R^4$ für einen $C_{1-10}$-Alkyl-Rest,

einen Aryl-Rest, einen Heteroaryl-Rest oder für einen über eine $C_{1-6}$-Alkylen-Gruppe gebundenen Aryl-Rest steht, kann nach der in Maruoka und Yamamoto, Angew. Chem., Vol. 97, pp. 670 -683, 1985, Maruoka et al. J. Am. Chem. Soc., Vol. 105, p. 2831, 1985 bzw. Maruoka et al. Org. Synth., Vol. 66, p. 185 angegebenen Methode durchgeführt werden. Die entsprechenden Offenbarungen werden hiermit als Referenz eingeführt.

Die Herstellung der Verbindungen der allgemeinen Formel I, worin der Rest $R^4$ für H, $SR^8$ oder $COCOR^5$ steht und die Reste $R^5$ und $R^8$ die Bedeutung gemäß der allgmeinen Formel I haben, kann nach den verschiedenen dem Fachmann bekannten Methoden erfolgen. Die Herstellung der Verbindungen der allgemeinen Formel I, worin der Rest $R^4$ für $CONR^6R^7$ oder $CSNR^6R^7$ steht und die Reste $R^6$ und $R^7$ entweder jeweils H bedeuten oder jeweils die Bedeutung der allgemeinen Formel I haben, jedoch ungleich H sind, kann ebenfalls nach den verschiedenen dem Fachmann bekannten Methoden erfolgen.

Untersuchungen mittels $^1$H-NMR-Spektroskopie zeigen, daß die nach den vorstehend genannten Verfahren erhaltenen Pyrrolidin-2,3,4-trion-Derivate . der allgemeinen Formel I als Gemisch von syn- und anti-Isomeren vorliegen können, die nicht weiter getrennt werden konnten.

Die erfindungsgemäßen Verbindungen der allgemeinen Formel I lassen sich mit Säuren, wie beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Asparaginsäure oder einem Gemisch aus wenigstens zwei dieser Säuren, in an sich bekannter Weise in die entsprechenden physiologisch verträglichen Salze überführen. Vorzugsweise wird die Salzbildung in einem Lösungsmittel, wie beispielsweise Diethylether, Diisopropylether, Essigsäurealkylester, Aceton, 2-Butanon oder einem Gemsich aus wenigstens zwei dieser Lösungsmittel durchgeführt. Zur Herstellung der entsprechenden Hydrochloride eignet sich darüber hinaus Trimethylchlorsilan in wäßriger Lösung.

Die erfindungsgemäßen substituierten Pyrrolidin-2,3,4-trion-3-oxim-Derivate der allgemeinen Formel I sind toxikologisch unbedenklich und stellen daher geeignete pharmazeutische Wirkstoffe dar.

Ein weiterer Gegenstand der Erfindung sind daher Arzneimittel, die als Wirkstoff wenigstens ein substituiertes Pyrrolidin-2,3,4-trion-Derivat der allgemeinen Formel I und/oder eine entsprechende Base und/oder ein entsprechendes physiologisch verträgliches Salz sowie gegebenenfalls weitere Wirk- und/oder Hilfsstoffe enthalten. Das Arzneimittel kann auch ein Gemisch aus wenigstens zwei Enantiomeren und/oder die entsprechenden Basen und/oder die entsprechenden physiologisch verträglichen Salze einer erfindungsgemäßen Verbindung der allgemeinen Formel I enthalten, wobei die Enantiomeren nicht in äquimolaren Mengen vorliegen.

Vorzugsweise werden die Arzneimittel zur Behandlung/Bekämpfung bei/von Schmerzen, inflammatorischen und/oder allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen, neurodegenerativen Erkrankungen, Epilepsie, Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Hirnödemen, Unterversorgungszuständen des zentralen Nervensystems, Hypoxie, Anoxie, AIDS-Demens, Encephalomyelitis, Tourette-Syndrom, perinataler Asphyxie oder zur Anxiolyse eingesetzt.

Ein weiterer Gegenstand der Erfindung ist auch die Verwendung von wenigstens einem substituierten Pyrrolidin-2,3,4-trion-Derivat der allgemeinen Formel I und/oder einer entsprechenden Base und/oder eines entsprechenden physiologisch verträglichen Salzes zur Herstellung eines Arzneimittels zur Behandlung/Bekämpfung bei/von Schmerzen, inflammatorischen und/oder allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen, neurodegenerativen Erkrankungen, Epilepsie, Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Hirnödemen, Unterversorgungszuständen des zentralen Nervensystems, Hypoxie, Anoxie, AIDS-Demens, Encephalomyelitis, Tourette-Syndrom, perinataler Asphyxie oder zur Anxiolyse.

Zur Zubereitung entsprechender pharmazeutischer Formulierungen werden neben mindestens einem substituierten Pyrrolidin-2,3,4-trion-Derivat der allgemeinen Formel I zusätzlich üblicherweise Hilfsstoffe, wie z.B. Trägermaterialien, Füllstoffe, Lösungsmittel, Verdünnungsmittel, Farbstoffe oder Bindemittel eingesetzt. Die Auswahl der Hilfsstoffe sowie die einzusetzenden Mengen derselben hängt von der Art der Applikation, wie z.B. oral, intravenös, intraperitoneal, intradermal, intramuskulär, intranasal, buccal oder örtlich, zum Beispiel auf Infektionen an der Haut, der Schleimhäute und an den Augen, ab und ist dem Fachmann bekannt. Für die orale Applikation eignen sich beispielsweise Zubereitungen in Form von Tabletten, Dragees, Kapseln, Granulaten, Tropfen, Säften und Sirupen sowie multipartikuläre Zubereitungen, beispielsweise Pellets oder Granulaten, die ggf. auch in Kapseln abgefüllt oder zu Tabletten verpreßt sein können, für die parenterale, topische und inhalative Applikation eignen sich z.B. Lösungen, Suspensionen, leicht rekonstituierbare Trockenzubereitungen sowie Sprays. Erfindungsgemäße Verbindungen der allgemeinen Formel I in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel I auch verzögert

freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 2 bis 500 mg/kg Körpergewicht des Patienten wenigstens eines Pyrrolidin-2,3,4-trion-3-oxim-Derivates der allgemeinen Formel I appliziert.

Pharmakologische Untersuchungen

a) Untersuchungen zur Rezeptorbindung:

Die Untersuchungen zur Bestimmung der Affinität der erfindungsgemäßen substituierten Pyrrolidin-2,3,4-trion-3-oxim-Derivate der allgemeinen Formel I zur Glycin-Bindungsstelle des NMDA-Rezeptorkanals wurde an Hirnmembranhomogenaten (Homogenat von Cortex- und Hippocampus-Areal aus dem Hirn von männlichen Ratten, Stamm Wistar, Charles River, WIGA GmbH, Sulzbach, Deutschland) nach Baron B.M. et al, J. Pharmacol. Exp. Ther., Vol. 279, pp. 62-68 (1996) durchgeführt.

Hierzu wurde Cortex und Hippocampus aus frisch entnommenen Rattengehirnen freipräpariert und in 5 mmol/l TRIS-Acetatpuffer, 0,32 mol/l Sacharose pH 7,4 (10 ml/g Frischgewicht) mit einem Potter-Homogenisator (Fa. Braun, Melsungen, Deutschland, 10 Kolbenhübe bei 500 Umdrehungen pro Minute (Upm)) unter Eiskühlung homogenisiert und anschließend für 10 Minuten bei 1.000 g und 4°C zentrifugiert. Der erste Überstand wurde gesammelt und das Sediment erneut mit 5 mmol/l TRIS-Acetatpuffer, 0,32 mol/l Sacharose pH 7,4 (5 ml/g ursprüngliche Frischeinwaage Rattenhirn-Cortex und Hippocampus) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wurde mit dem Überstand aus der ersten Zentrifugation vereinigt und bei 17.000 g für 20 Minuten bei 4°C zentrifugiert. Der Überstand nach dieser Zentrifugation wurde verworfen und das Membransediment mit 5 mmol/l TRIS-Acetatpuffer pH 8,0 (20 ml/g ursprüngliches Frischgewicht) aufgenommen und mit 10 Kolbenhüben bei 500 Upm homogenisiert.
Anschließend wurde das Membranhomogenat für 1 Stunde bei 4°C inkubiert und für 30 Minuten bei 50 000 g und 4°C zentrifugiert. Der Überstand wurde verworfen und das Zentrifugenröhrchen mit dem Membransediment mit Parafilm verschlossen und für 24 Stunden bei -20°C eingefroren. Anschließend wurde das Membransediment aufgetaut und mit eiskaltem 5 mmol/l TRIS-Acetatpuffer, 0,1 % Saponin (Gewicht/Volumen) pH 7,0 (10 ml/g ursprüngliches Frischeinwaage) aufgenommen und mit 10 Kolbenhüben bei 500 Upm homogenisiert und anschließend für 20 Minuten bei 50.000 g und 4°C zentrifugiert. Der resultierende Überstand wurde verworfen und das Sediment in einem kleinen Volumen mit 5 mmol/l TRIS-Acetatpuffer pH 7,0 (ca. 2 ml/g ursprüngliches Frischgewicht) aufgenommen und erneut mit 10 Kolbenhüben bei 500 Upm homogenisiert. Nach Bestimmung des Proteingehaltes wurde das Membranhomogenat mit 5 mmol/l TRIS-Acetatpuffer pH 7,0 auf eine Proteinkonzentration von 10 mg Protein/ml eingestellt und in Aliquoten bis zur Durchführung der Untersuchung eingefroren.

Für den Rezeptorbindungstest wurden Aliquote aufgetaut, 1:10 mit 5 mmol/) TRIS-Acetatpuffer pH 7,0 verdünnt, mit 10 Kolbenhüben bei 500 Upm mit dem Potter-Homogenisator unter Eiskühlung homogenisiert und für 60 Minuten bei 55.000 g bei 4°C zentrifugiert. Der Überstand wurde dekantiert und das Membransediment mit eiskaltem 50 mmol/l TRIS-Acetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Von diesem Membranhomogenat wurden jeweils 100 µl je 1 ml-Ansatz im Rezeptorbindungstest eingesetzt (0,1 mg Protein/ml im Endansatz).

Im Bindungstest wurde als Puffer 50 mmol/l TRIS-Acetatpuffer pH 7,0 sowie als radioaktiver Ligand 1 nmol/l ($^3$H)-MDL 105.519 (Baron B.M. et al, J. Pharmacol. Exp. Ther., Vol. 279 , pp. 62-68 (1996)) eingesetzt. Der Anteil an unspezifischer Bindung wurde in Anwesenheit von 1 mmol/l Glycin bestimmt.

In weiteren Ansätzen wurden die erfindungsgemäßen Verbindungen in Konzentrationsreihen zugegeben und die Verdrängung des radioaktiven Liganden aus seiner spezifischen Bindung an die Glycin-Bindungsstelle des NMDA-Rezeptorkanals ermittelt. Die jeweiligen Dreifachansätze wurden über 120 Minuten bei 4°C inkubiert und anschließend zur Bestimmung des an das Membranhomogenat gebundenen radioaktiven Liganden mittels Filtration durch Glasfaser-Filtermatten (Typ Whatman GF/B, Fa. Adi Hassel, München, Deutschland) geerntet. Die auf den Glasfaser-Filtern zurückgehaltene Radioaktivität wurde nach Zugabe von Szintillator (Ready Protein, Fa. Beckamnn Coulter GmbH, Krefeld, Deutschland) im ß-Counter (Packard TRI-CARB Liquid Szintillation Analyzer 2000CA, Fa. Packard Instrument, Meriden, CT 06450, USA) gemessen.

Die Affinität der erfindungsgemäßen Verbindungen zur Glycin-Bindungsstelle des NMDA-Rezeptorkanals wurde als $IC_{50}$ (Konzentration mit 50 % Verdrängung des radioaktiven Liganden aus seiner spezifischen Bindung) nach dem Massenwirkungsgesetz mittels nichtlinearer Regression berechnet und nach Umrechnung (nach der Cheng-Prussoff-Gleichung (Y. Cheng, W.H. Prusoff, 1973, Biochem. Pharmacol., Vol. 22, pp. 3099-3108)) als Ki-Wert angegeben.

b) NMDA/Glycin-induzierte Ionenströme an RNA-injizierten Xenopus Oocyten

Die Untersuchung zur Bestimmung von Funktionsänderungen des NMDA-Rezeptorkanals durch die erfindungsgemäßen Verbindungen der allgemeinen Formel I wurde an Oozyten des Südafrikanischen Krallenfrosches, Xenopus laevis, durchgeführt. Hierzu wurden neuronale NMDA-Rezeptorkanäle nach Injektion von RNA aus Mäusehirnen in Oozyten ausgebildet und durch Koapplikation von NMDA und Glycin ausgelöste Ionenströme gemessen.
Xenopus Oozyten der Stadien V und VI (Dumont, J.N., J. Morphol., Vol. 136, pp. 153-180 (1972)) wurden mit Gesamt-RNA aus Hirngewebe adulter Mäuse mikroinjiziert (100-130 ng/Zelle) und bis zu 10 Tage in Kulturmedium (Zusammensetzung: 88.0 mmol/l NaCl, 1.0 mmol/l KCl, 1.5 mmol/l $CaCl_2$, 0.8 mmol/l $MgSO_4$, 2.4 mmol/l $NaHCO_3$, 5 mmol/l HEPES, 100 IU/ml Penicillin, 100 µg/ml Streptomycin, pH 7.4) bei 20 °C gehalten. Transmembranöse Ionenströme wurden mit Hilfe der konventionellen Zwei-Elektroden-Spannungsklemmtechnik bei einem Haltepotential von -70 mV registriert (Bloms-Funke P. et al, (1996) Neurosci. Lett. 205, pp. 115-118 (1996)). Zur Datenaufzeichnung und Steuerung der Versuchsapparatur wurden das OTC-Interface und die Software Cellworks verwendet (Firma npi, Bundesrepublik Deutschland). Die erfindungsgemäßen Verbindungen wurden einem nominal $Mg^{2+}$freien Medium (Zusammensetzung: 89.0 mmol/l NaCl, 1.0 mmol/l KCl, 1.8 mmol/l $CaCl_2$, 2.4 mmol/l $NaHCO_3$, 5 mmol/l HEPES, pH 7.4) zugesetzt und mit Hilfe einer Konzentrationsklemme systemisch appliziert (Firma npi, Bundesrepublik Deutschland). Um Substanzeffekte zu testen, die über die Glycin B-Bindungsstelle des NMDA-Rezeptorkanals vermittelt sind, wurde die Glycin-Dosiswirkungskurve mit und ohne die jeweilige erfindungsgemäße Verbindung aufgezeichnet. Dazu wurde NMDA in einer fixen Konzentration von 100 µmol/l mit Glycin in steigenden Konzentrationen (0-100 µmol/l) kumulativ koappliziert. Im Anschluß wurde das Experiment in gleicher Weise mit einer festen Konzentration der erfindungsgemäßen Verbindung wiederholt. Die Stromamplituden wurden auf die der Kontrollantwort auf Koapplikation von NMDA (100 µmol/l) mit Glycin (10 µmol/l) normiert. Die Analyse der Daten wurde mit der Software Igor-Pro (Version 3.1, WaveMetrics, USA) durchgeführt. Alle Ergebnisse wurden als Mittelwert aus mindestens 3 Experimenten an verschiedenen Oozyten von mindestens zwei Fröschen angegeben. Die Signifikanz für ungepaarte Meßgrößen wird mit Hilfe des Mann-Whitney U-Test und für gepaarte Meßgrößen durch den Wilcoxon-Test ermittelt (Sysstat, SPSS Inc., USA). $EC_{50}$-Werte werden nach folgender Formel berechnet:

$$Y = Y_{min} + (Y_{max} - Y_{min}) / (1 + (X/EC_{50})^{-p})$$

(**$Y_{min}$** = minimaler Testwert, **$Y_{max}$** = maximaler Testwert, **Y**= relative Stromamplitude, **X** = Konzentration der Testsubstanz, **p** = Slope-Faktor). Bei Rechtsverschiebung der Glycin-Dosiswirkungskurve wurde anhand einer Schild-Regression der $pA_2$-Wert der erfindungsgemäßen Verbindung graphisch ermittelt. Konzentrationsverhältnisse wurden anhand der $EC_{50}$-Werte kalkuliert, die für jede Dosiswirkungskurve unabhängig errechnet wurden.

c) Formalin-Test an der Maus

Die Untersuchungen zur Bestimmung der antinociceptiven Wirkung der erfindungsgemäßen Verbindungen wurden im Formalin-Test an männlichen Albino-Mäusen (NMRI, 25 - 35 g, Iffa Credo, Belgien) durchgeführt.
Im Formalin-Test werden die erste (frühe) Phase (0 - 15 min nach Formalin-Injektion) und die zweite (späte) Phase (15-60 min nach Formalin-Injektion) unterschieden (D. Dubuisson er al, Pain, Vol. 4, pp. 161 - 174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T.J. Coderre et al, Pain, Vol. 52, pp. 259 - 285 (1993)).
Die erfindungsgemäßen Verbindungen wurden in der zweiten Phase des Formalin-Tests untersucht, um Aussagen über die Wirkungen der erfindungsgemäßen Verbindungen im chronisch/entzündlichen Schmerz zu erhalten. Durch eine einmalige subkutane Formalin-Injektion (20 µl, 1 %ige wäßrige Lösung) in die dorsale Seite der rechten Hinterpfote wurde bei freibeweglichen Versuchstieren eine nociceptive Reaktion induziert, die sich in deutlichem Lekken und Beißen der betroffenen Pfote äußert.
Für den Untersuchungszeitraum in der zweiten (späten) Phase des Formalin-Tests wurde das nozizeptive Verhalten durch Beobachtung der Tiere kontinuierlich erfaßt. Die Quantifizierung des Schmerzverhaltens erfolgte durch Summation der Sekunden, in denen die Tiere im Untersuchungszeitraum Lecken und Beißen der betroffenen Pfote zeigten. Nach Injektion von Verbindungen, die im Formalin-Test antinozizeptiv wirksam sind, sind die beschriebenen Verhaltensweisen der Tiere reduziert, evtl. sogar aufgehoben. Entsprechend den Versuchen, bei denen die Tiere die erfindungsgemäßen Verbindungen vor der Applikation von Formalin injiziert bekommen hatten, wurde den Kontrolltieren Vehikel, d.h. Lösungmittel (z.B. 0,9%ige NaCl-Lösung), vor der Formalin-applikation gespritzt. Das Verhalten der Ratten nach Substanzgabe (10 Mäuse pro Substanzdosierung) wurde mit einer Kontrollgruppe (10 Mäuse) verglichen. Basierend auf der Quantifizierung des Schmerzverhaltens wurde die Substanzwirkung im Formalin-Test als Änderung

der Kontrolle in Prozent ermittelt. Die $ED_{50}$-Berechnungen erfolgten mittels Regressionsanalyse. Abhängig von der Applikationsart der erfindungsgemäßen Verbindungen wurde der Applikationszeitpunkt vor der Formalin-Injektion gewählt (intraperitoneal: 15 min, intravenös: 5 min).

d) Writhing-Test an der Maus

Die Untersuchung auf analgetische Wirksamkeit wurde auch im Phenylchinon-induzierten Writhing an der Maus (modifiziert nach I.C. Hendershot et al, (1959) J. Pharmacol. Exp. Ther., Vol. 125, pp. 237-240) durchgeführt. Dazu wurden männliche NMRI-Mäuse im Gewicht von 25 bis 30 g (Iffa, Credo, Belgien) verwendet. Gruppen von 10 Tieren pro Substanzdosis erhielten 10 Minuten nach intravenöser Gabe der jeweiligen Verbindung 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der Schmerz-induzierten Streckbewegungen (sogenannte Writhing-reaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten haben. Alle Substanzen wurden in der Standarddosierung von 10 mg/kg Körpergewicht Maus getestet. Die prozentuale Hemmung (%Hemmung) der Writhingreaktion durch eine Substanz wurde nach folgender Formel berechnet:

$$\%\ \text{Hemmung} = 100 - \frac{\text{Writhingreaktionen der behandelten Tiere}}{\text{Writhingreaktionen der Kontrolltiere}} * 100$$

Für einige der erfindungsgemäßen Verbindungen wurde aus der dosisabhängigen Abnahme der Writhingreaktionen im Vergleich zu parallel untersuchten Phenylchinon-Kontrollgruppen mittels Regressions-analyse (Auswerteprogramm Martens EDV Service, Eckental) die $ED_{50}$-Werte mit 95 % Vertrauensbereich der Writhingreaktion berechnet.

Die folgenden Beispiele dienen der Erläuterung der Erfindung, schränken aber den allgemeinen Erfindungsgedanken nicht ein.

**Beispiele:**

Die Ausbeuten der hergestellten Verbindungen sind nicht optimiert.

Die bestimmten Schmelzpunkte sind unkorrigiert.

Beispiel 1:

5-(Methoxyphenylmethylen)-pyrrolidin-2,3,4-trion-3-oxim

Zu einer eisgekühlten Lösung von 2 mmol 4-Hydroxy-5-(methoxyphenyl-methylen)-1,5-dihydropyrrol-2-on (hergestellt nach H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmot (0,075 g) Natriumnitrit zugetropft. Die resultierende Lösung wurde dann 30 Minuten bei Raumtemperatur gerührt und im Vakuum eingeengt. Der Rückstand wird durch Umkristallisation aus Ethanol gereinigt. Die Ausbeute an 5-(Methoxyphenylmethylen)-pyrrolidin-2,3,4-trion-3-oxim betrug 60 % mit einem Schmelzpunkt von 174°C.

Die Untersuchung dieser Verbindungen mittels $^1$H-NMR-Spektroskopie ergab die folgenden Signale: (d6-DMSO, δ in ppm): 11,95 (s, 1 H); 10,74 (s, 0,66 H); 10,71 (s, 0,33 H); 7,46 (m, 5 H); 3,42 (s, 1 H); 3,41 (s, 2 H).

Beispiel 2:

5-(Bromphenylmethylen)-pyrrolidin-2,3,4-trion-3-oxim

Zu einer eisgekühlten Lösung von 2 mmol 5-(Bromophenyl-methylen)-4-hydroxy-1,5-dihydropyrrol-2-on (hergestellt nach H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmol (0,075 g) Natriumnitrit zugetropft. Die resultierende Lösung wurde dann 30 Minuten bei-Raumtemperatur gerührt und im Vakuum eingeengt. Der Rückstand wird durch Umkristallisation aus Ethanol gereinigt. Die Ausbeute an 5-(Bromphenylmethylen)-pyrrolidin-2,3,4-trion-3-oxim betrug 65 % mit

einem Schmelzpunkt von 158°C.

Die Untersuchung dieser Verbindungen mittels $^1$H-NMR-Spektroskopie ergab die folgenden Signale: (d6-DMSO, δ in ppm): 14,46 (s, 1 H); 11,06 (s, 0,70 H); 11,00 (s, 0,30 H); 7,40-7,26 (m, 5 H).

Beispiel 3:

5-Benzyliden-pyrrolidin-2,3,4-trion-3-oxim

Zu einer eisgekühlten Lösung von 2 mmol 5-Benzyliden-4-hydroxy-1,5-dihydropyrrol-2-on (hergestellt nach H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmol (0,075 g) Natriumnitrit zugetropft. Die resultierende Lösung wurde dann 30 Minuten bei Raumtemperatur gerührt und im Vakuum eingeengt. Der Rückstand wird durch Umkristallisation aus Ethanol gereinigt. Die Ausbeute an 5-Benzyliden-pyrrolidin-2,3,4-trion-3-oxim betrug 65 % mit einem Schmelzpunkt von 205°C.

Die Untersuchung dieser Verbindungen mittels $^1$ H-NMR-Spektroskopie ergab die folgenden Signale: (d6-DMSO, δ in ppm): 14,66 (s, 1 H); 11,25 (s, 0,66 H); 11,18 (s, 0,33 H); 7,64-7,31 (m, 5 H); 6,42 (s, 0,33 H); 6,36 (s, 0,66 H).

Beispiel 4:

5-(2-Chlorbenzyliden)-pyrrolidin-2,3,4-trion-3-oxim

Zu einer eisgekühlten Lösung von 2 mmol 5-(2-Chlorbenzyliden)-4-hydroxy-1,5-dihydropyrrol-2-on (hergestellt nach H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmol (0,075 g) Natriumnitrit zugetropft. Die resultierende Lösung wurde dann 30 Minuten bei Raumtemperatur gerührt und im Vakuum eingeengt. Der Rückstand wird durch Umkristallisation aus Ethanol gereinigt. Die Ausbeute an 5-(2-Chlorbenzyliden)-pyrrolidin-2,3,4-trion-3-oxim betrug 60 % mit einem Schmelzpunkt von 176°C.

Die Untersuchung dieser Verbindungen mittels $^1$ H-NMR-Spektroskopie ergab die folgenden Signale: (d6-DMSO, δ in ppm): 11,40 (s, 0,66 H); 11,34 (s, 0,33 H);
7,46-7,21 (m, 4 H); 6,52 (s, 0,33 H); 6,47 (s, 0,66 H).

Beispiel 5:

5-(4-Chlorbenzyliden)-pyrrolidin-2,3,4-trion-3-oxim

Zu einer eisgekühlten Lösung von 2 mmol 5-(4-Chlorbenzyliden)-4-hydroxy-1,5-dihydropyrrol-2-on (hergestellt nach H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmol (0,075 g) Natriumnitrit zugetropft. Die resultierende Lösung wurde dann 30 Minuten bei Raumtemperatur gerührt und im Vakuum eingeengt. Der Rückstand wird durch Umkristallisation aus Ethanol gereinigt. Die Ausbeute an 5-(4-Chlorbenzyliden)-pyrrolidin-2,3,4-trion-3-oxim betrug 50 % mit einem Schmelzpunkt von 190°C.

Die Untersuchung dieser Verbindungen mittels $^1$ H-NMR-Spektroskopie ergab die folgenden Signale: (d6-DMSO, δ in ppm): 11,35 (s, 0,66 H); 11,28 (s, 0,33 H);
7,67-7,64 (m, 2 H); 7,45-7,35 (m, 2 H); 6,40 (s, 0,33 H); 6,35 (s, 0,66 H).

Beispiel 6:

5-(2,3-Dichlorbenzyliden)-pyrrolidin-2,3,4-trion-3-oxim

Zu einer eisgekühlten Lösung von 2 mmol 5-(2,3-Dichlorbenzyliden)-4-hydroxy-1,5-dihydropyrrol-2-on (hergestellt nach H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmol (0,075 g) Natriumnitrit zugetropft. Die resultierende Lösung wurde dann 30 Minuten bei Raumtemperatur gerührt und im Vakuum eingeengt. Der Rückstand wird durch Umkristallisation aus Ethanol gereinigt. Die Ausbeute an 5-(2,4-Dichlorbenzyliden)-pyrrolidin-2,3,4-trion-3-oxim betrug 55 % mit einem Schmelzpunkt von 180 °C.

Die Untersuchung dieser Verbindungen mittels $^1$H-NMR-Spektroskopie ergab die folgenden Signale: (d6-DMSO, δ in ppm): 11,43 (s, 0,66 H); 11,37 (s, 0,33 H); 7,63-7,37 (m, 3 H); 6,50 (s, 0,33 H); 6,45 (s, 0,66 H).

Beispiel 7:

5-(2,4-Dichlorbenzyliden)-pyrrolidin-2,3,4-trion-3-oxim

Zu einer eisgekühlten Lösung von 2 mmol 5-(2,4-Dichlorbenzyliden)-4-hydroxy-1,5-dihydropyrrol-2-on (hergestellt nach. H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmol (0,075 g) Natriumnitrit zugetropft. Die resultierende Lösung wurde dann 30 Minuten bei Raumtemperatur gerührt und im Vakuum eingeengt. Der Rückstand wird durch Umkristallisation aus Ethanol gereinigt. Die Ausbeute an 5-(2,4-Dichlorbenzyliden)-pyrrolidin-2,3,4-trion-3-oxim betrug 55 % mit einem Schmelzpunkt von 180°C.

Die Untersuchung dieser Verbindungen mittels $^1$H-NMR-Spektroskopie ergab die folgenden Signale: (d6-DMSO, δ in ppm): 11,38 (s, 0,66 H); 11,32 (s, 0,33 H); 7,68-7,65 (m, 2H); 7,45-7,43 (m, 1 H); 6,42 (s, 0,33 H); 6,36 (s, 0,66 H).

Beispiel 8:

5-(2,6-Dichlorbenzyliden)-pyrrolidin-2,3,4-trion-3-oxim

Zu einer eisgekühlten Lösung von 2 mmol 5-(2,6-Dichlorbenzyliden)-4-hydroxy-1,5-dihydropyrrol-2-on (hergestellt nach H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmol (0,075 g) Natriumnitrit zugetropft. Die resultierende Lösung wurde dann 30 Minuten bei Raumtemperatur gerührt und im Vakuum eingeengt. Der Rückstand wird durch Umkristallisation aus Ethanol gereinigt. Die Ausbeute an 5-(2,6-Dichlorbenzyliden)-pyrrolidin-2,3,4-trion-3-oxim betrug 65 % mit einem Schmelzpunkt von 232 °C.

Die Untersuchung dieser Verbindungen mittels $^1$H-NMR-Spektroskopie ergab die folgenden Signale: (d6-DMSO, δ in ppm): 11,12 (s, 0,66 H); 11,08 (s, 0,33 H); 7,50-7,48 (m, 2 H); 7,39-7,35 (m, 1 H); 6,27 (s, 0,33 H); 6,22 (s, 0,66 H).

Beispiel 9:

5-(3-Chlorbenzyliden)-pyrrolidin-2,3,4-trion-3-oxim

Zu einer eisgekühlten Lösung von 2 mmol 5-(3-Chlorbenzyliden)-4-hydroxy-1,5-dihydropyrrol-2-on (hergestellt nach H. Poschenrieder et al (Arch. Pharm. Pharm. Med. Chem. 1998, 331, 389-394) und H.-D. Stachel et al (J. Heterocycl. Chem. 1980, Vol. 17, pp. 1195-1199 und Liebigs Ann. Chem. 1985, pp. 1692-1696)) in 5 ml Eisessig wurde unter Rühren eine wäßrige Lösung von 1,1 mmol (0,075 g) Natriumnitrit zugetropft. Die resultierende Lösung wurde dann 30 Minuten bei Raumtemperatur gerührt und im Vakuum eingeengt. Der Rückstand wird durch Umkristallisation aus Ethanol gereinigt. Die Ausbeute an 5-(3-Chlorbenzyliden)-pyrrolidin-2,3,4-trion-3-oxim betrug 50 % mit einem Schmelzpunkt von 185°C.

Die Untersuchung dieser Verbindungen mittels $^1$H-NMR-Spektroskopie ergab die folgenden Signale: (d6-DMSO, δ in ppm): 14,69 (s, 0,66 H); 14,53 (s, 0,33 H); 11,47 (s, 0,66 H), 11,40 (s, 0,33 H); 7,72-7,20 (m, 4 H); 6,38 (s, 0,33 H); 6,33 (s, 0,66 H).

Pharmakologische Untersuchungen

a) Untersuchungen zur Rezeptorbindung

Die. Untersuchungen zur Bestimmung der Affinität der erfindungsgemäßen Verbindungen gemäß Beispiel 1 und 2 zur Glycin-Bindungsstelle des NMDA-Rezeptorkanals wurden wie öbenstehend beschrieben durchgeführt.

Die Affinität Glycin-Bindungsstelle des NMDA-Rezeptorkanals wurde als $IC_{50}$ (Konzentration mit 50 % Verdrängung des radioaktiven Liganden aus seiner spezifischen Bindung) nach dem Massenwirkungsgesetz mittels nichtlinearer Regression berechnet und ist in der nachstehenden Tabelle 1; nach Umrechnung (nach der Cheng-Prussoff-Gleichung (Y. Cheng, W.H. Prusoff, 1973, Biochem. Pharmacol., Vol. 22, pp. 3099-3108)) als Ki-Wert angegeben.

Tabelle 1

| Beispiel | Glycin-Bindungsstelle des NMDA-Rezeptorkanals Ki (μmol/l). |
|---|---|
| 1 | 0,116 |
| 2 | 0,430 |

b) NMDA/Glycin-induzierte Ionenströme an RNA-injizierten Xenopus Oocyten

Die Untersuchung zur Bestimmung von Funktionsänderungen des NMDA-Rezeptorkanals durch die erfindungsgemäße Verbindung gemäß Beispiel 1 wurde wie obenstehend beschrieben durchgeführt.

Das Ergebnis zur Untersuchung der Wirkung der erfindungsgemäßen Verbindung gemäß Beispiel 1 auf durch NMDA/Glycin ausgelöste Ionenströme an RNA-' injizierten Oozyten ist in der nachstehenden Tabelle 2 angegeben.

Tabelle 2:

| Beispiel Nr. | NMDA-induzierte relative Stromamplitude n | Ionenströme | |
|---|---|---|---|
| | NMDA | NMDA + Glycin (0.3 μM) | NMDA + Glycin (10 μM) |
| Kontrolle<br>Beispiel 1 | 1.42%.<br>-0.58 % | 70.23%<br>0.08 % | 100 %<br>59.93 % |

Aus den Untersuchungen ergibt sichdie antagonistische Wirkung der Verbindung gemäß Beispiel 1.

c) Formalin-Test an der Maus

Die Untersuchungen zur Bestimmung der antinociceptiven Wirkung der erfindungsgemäßen Verbindungen wurde wie obenstehend beschrieben durchgeführt.

Die entsprechenden Ergebnisse im Formalin-Test an der Maus sind in der nachfolgenden Tabelle 3 zusammengefaßt.

Tabelle 3:

| Beispiel | % Änderung gegen Kontrolle bei 10 mg/kg |
|---|---|
| 1 | 63,9 |
| 2 | 36,3 |
| 3 | 47,6 |

d) Writhing-Test an der Maus

Die Untersuchung auf analgetische Wirksamkeit wurde im Phenylchinon-induzierten Writhing an der Maus wie obenstehend beschrieben durchgeführt. Alle untersuchten erfindungsgemäßen Verbindungen zeigten eine ausgeprägte analgetische Wirkung. Die Ergebnisse sind in der nachfolgenden Tabelle 4 zusammengefaßt.

Tabelle 4:

| Beispiel | %Hemmung der Writhingreaktion bei 10 mg/kg intravenös |
|---|---|
| 3 | 25 |
| 4 | 55 |
| 5 | 50 |
| 6 | 52 |
| 7 | 46 |

Tabelle 4: (fortgesetzt)

| Beispiel | %Hemmung der Writhingreaktion bei 10 mg/kg intravenös |
|---|---|
| 8 | 51 |
| 9 | 81 |

**Patentansprüche**

1. Substituierte Pyrrolidin-2,3,4-trion-Derivate der allgemeinen Formel I,

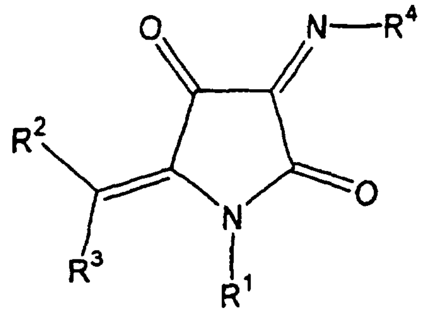

I

worin
der Rest $R^1$ für H, $OR^8$, $COR^5$, $CSR^5$, $NR^6R^7$, $COOR^5$, $CONR^6R^7$, $CSNR^6R^7$, einen $C_{1-10}$-Alkyl- oder einen unsubstituierten Phenyl-Rest steht,
die Reste $R^2$, $R^3$, gleich oder verschieden, für H, F, Cl, Br, $CF_3$, $OR^8$, $SR^8$, einen $C_{1-10}$-Alkyl-, einen Aryl-, einen Heteroaryl-Rest oder für einen über eine $C_{1-6}$-Alkylen-Gruppe gebundenen Aryl-Rest stehen,
der Rest $R^4$ für H, OH, $OR^8$, $SR^8$, $COR^5$, $COOR^5$, $COCOR^5$, $CONR^6R^7$, $CSNR^6R^7$ oder einen $C_{1-10}$-Alkyl-Rest steht,
der Rest $R^5$ für H oder einen $C_{1-10}$-Alkyl-Rest steht,
die Reste $R^6$, $R^7$, gleich oder verschieden, für H, $OR^8$, $COR^5$, $COOR^5$, oder einen $C_{1-10}$-Alkyl-Rest stehen,
der Rest $R^8$ für einen $C_{1-10}$-Alkyl-Rest steht,
in Form ihrer Racemate, Enantiomere, Diastereomere oder einer entsprechenden Base oder eines entsprechenden physiologisch verträglichen Salzes.

2. Substituierte Pyrrolidin-2,3,4-trion-Derivate gemäß Anspruch 1, **dadurch gekennzeichnet, daß** der Rest $R^1$ für einen $C_{1-6}$-Alkylrest steht und die übrigen Reste $R^2$ bis $R^8$ die Bedeutung gemäß der allgemeinen Formel I haben.

3. Substituierte Pyrrolidin-2,3,4-trion-Derivate gemäß Anspruch 1 oder 2. **dadurch gekennzeichnet, daß** der Rest $R^2$ und/oder $R^3$ für einen $C_{1-6}$-Alkylrest steht und die übrigen Reste $R^4$ bis $R^8$ sowie ggf. $R^2$ oder $R^3$ die Bedeutung gemäß der allgemeinen Formel I haben.

4. Substituierte Pyrrolidin-2,3,4-trion-Derivate gemäß Anspruch 1 oder 2, **dadurch gekennzeichnet, daß** der Rest $R^2$ und/oder $R^3$ für einen über eine $C_{1-3}$-Alkylen-Gruppe gebundenen Aryl-Rest steht und die übrigen Reste $R^4$ bis $R^8$ sowie ggf. $R^2$ oder $R^3$ die Bedeutung gemäß der allgemeinen Formel I haben.

5. Substituierte Pyrrolidin-2,3,4-trion-Derivate gemäß einem der Ansprüche 1 bis 4, **dadurch gekennzeichnet, daß** der Rest $R^4$ für OH steht und die übrigen Reste $R^5$ bis $R^8$ die Bedeutung gemäß der allgemeinen Formel I haben.

6. Substituierte Pyrrolidin-2,3,4-trion-Derivate gemäß einem der Ansprüche 1 bis 4, **dadurch gekennzeichnet, daß** der Rest $R^4$ für $OR^8$ steht und die übrigen Reste $R^5$ bis $R^8$ die Bedeutung gemäß der allgemeinen Formel I haben.

7. Substituierte Pyrrolidin-2,3,4-trion-Derivate gemäß einem der Ansprüche 1 bis 4, **dadurch gekennzeichnet, daß** der Rest $R^4$ für einen $C_{1-6}$-Alkyl-Rest steht und die übrigen Reste $R^5$ bis $R^8$ die Bedeutung gemäß der allgemeinen Formel I haben.

8. Substituierte Pyrrolidin-2,3,4-trion-Derivate gemäß einem der Ansprüche 1 bis 7, **dadurch gekennzeichnet, daß** der Rest $R^5$ für einen $C_{1-6}$-Alkyl - Rest steht und die übrigen Reste $R^6$ bis $R^8$ die Bedeutung gemäß der allgemeinen Formel I haben.

9. Substituierte Pyrrolidin-2,3,4-trion-Derivate gemäß einem der Ansprüche 1 bis 8, **dadurch gekennzeichnet, daß** der Rest $R^6$ und/oder $R^7$ für einen $C_{1-6}$-Alkylrest steht und der Rest $R^8$ sowie ggf. $R^6$ oder $R^7$ die Bedeutung gemäß der allgemeinen Formel I hat.

10. Substituierte Pyrrolidin-2,3,4-trion-Derivate gemäß einem der Ansprüche 1 bis 9, **dadurch gekennzeichnet, daß** der Rest $R^8$ für einen $C_{1-6}$-Alkyl - Rest steht.

11. Substituierte Pyrrolidin-2,3,4-trion-3-oxim-Derivate gemäß Anspruch 1
5-(Methoxyphenylmethylen)-pyrrolidin-2,3,4-trion-3-oxim,
5-(Bromphenylmethylen)-pyrrolidin-2,3,4-trion-3-oxirn.

12. Verfahren zur Herstellung von substituierten Pyrrolidin-2,3,4-trion-Derivaten der allgemeinen Formel I gemäß Anspruch 1, **dadurch gekennzeichnet, daß** man Tetramsäuren der allgemeinen Formel II

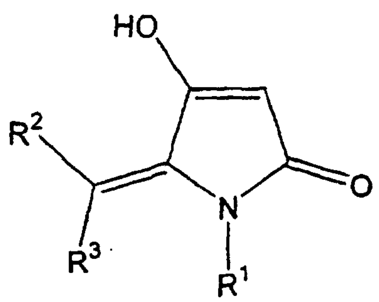

II

worin die Reste $R^1$ bis $R^3$ die Bedeutung gemäß der allgemeinen Formel I haben, in eisgekühlter Lösung mit einer wäßrigen Lösung von Natriumnitrit zu Verbindungen der allgemeinen Formel I, worin der $R^4$ für OH steht und die Reste $R^1$ bis $R^3$ die Bedeutung gemäß der allgemeinen Formel I haben, umsetzt, und diese reinigt und isoliert.

13. Verfahren zur Herstellung von substituierten Pyrrolidin-2,3,4-trion-Derivaten der allgemeinen Formel I gemäß Anspruch 1, **dadurch gekennzeichnet, daß** man die Verbindungen der allgemeinen Formel I worin der Rest $R^4$ für OH steht und die Reste $R^1$ bis $R^3$ die Bedeutung gemäß der allgemeinen Formel I haben in absoluten Lösungsmitteln bei niedrigen Temperaturen in Gegenwart von starken Basen mit $C_{1-10}$-Alkylhalogeniden zu Verbindungen der allgemeinen Formel I umsetzt, worin der Rest $R^4$ für $OR^8$ steht und die Reste $R^1$ bis $R^3$ sowie $R^8$ die Bedeutung gemäß der allgemeinen Formel I haben.

14. Verfahren zur Herstellung von substituierten Pyrrolidin-2,3,4-trion-Derivaten der allgemeinen Formel I gemäß Anspruch 1, **dadurch gekennzeichnet, daß** man die Verbindungen der allgemeinen Formel I, worin der Rest $R^4$ für $OR^8$ steht und die Reste $R^1$ bis $R^3$ sowie $R^8$ die Bedeutung gemäß der allgemeinen Formel I haben, in einem absoluten Lösungsmittel mit Säurechloriden der allgemeinen Formel $R^5$-(C=O)-Cl und/oder Säurebromiden der allgemeinen Formel $R^5$-(C=O)-Br oder Chlorameisensäureestern der allgemeinen Formel Cl-(C=O)-O-$R^5$ oder Fluorameisensäureestern der allgemeinen Formel F-(C=O)-O-$R^5$ oder mit öffenkettigen Carbonaten der allgemeinen Formel $R^5$-O-(C=O)-O-$R^5$ oder mit entsprechend substituierten cyclischen Carbonaten, worin jeweils der Rest R5 die Bedeutung gemäß der allgemeinen Formel I hat, zu Verbindungen der allgemeinen Formel I, worin der Rest $R^4$ für $COR^5$ oder $COOR^5$ steht und die Reste $R^1$ bis $R^3$ sowie $R^5$ die Bedeutung gemäß der allgemeinen Formel I haben, umsetzt, und nach den üblichen Verfahren reinigt und isoliert.

**15.** Verfahren zur Herstellung von substituierten Pyrrolidin-2,3,4-trion-Derivaten der allgemeinen Formel I gemäß Anspruch 1, **dadurch gekennzeichnet, daß** man die Verbindungen der allgemeinen Formel I, worin der Rest $R^4$ für OH steht und die Reste $R^1$ bis $R^3$ die Bedeutung gemäß der allgemeinen Formel I haben, mit aliphatischen Isocyanaten oder Isothiocyanaten bei niedrigen Temperaturen in aprotischen, polaren Lösungsmitteln zu Verbindungen der allgemeinen Formel I, worin $R^4$ für $CONR^6R^7$ oder $CSNR^6R^7$ steht, der Rest $R^6$ oder $R^7$ H bedeutet und die Reste $R^1$ bis $R^3$ sowie $R^6$ oder $R^7$ die Bedeutung gemäß der allgemeinen Formel I haben, umsetzt, und nach den üblichen Verfahren reinigt und isoliert.

**16.** Verfahren nach Anspruch 12, **dadurch gekennzeichnet, daß** man die Tetramsäuren der allgemeinen Formel II in einer eisgekühlten Lösung von Eisessig mit einer wäßrigen Lösung von Natriumnitrit umsetzt.

**17.** Verfahren nach Anspruch 12 oder 16, **dadurch gekennzeichnet, daß** die Verbindungen der allgemeinen Formel I, worin der Rest $R^4$ für OH steht und die Reste $R^1$ bis $R^3$ die Bedeutung gemäß der allgemeinen Formel I haben, durch Umkristallisation, vorzugsweise durch Umkristallisation aus Ethanol gereinigt und isoliert werden.

**18.** Verfahren nach Anspruch 13, **dadurch gekennzeichnet, daß** man die Verbindungen der allgemeinen Formel I, worin $R^4$ für OH steht und die Reste $R^1$ bis $R^3$ die Bedeutung gemäß der allgemeinen Formel I haben, unter Schutzgasatmosphäre umsetzt.

**19.** Verfahren nach Anspruch 13 oder 18, **dadurch gekennzeichnet, daß** man die Verbindungen der allgemeinen Formel I, worin $R^4$ für OH steht und die Reste $R^1$ bis $R^3$ die Bedeutung gemäß der allgemeinen Formel I haben, in offenkettigen und/oder cyclischen Ethem umsetzt.

**20.** Verfahren nach einem der Ansprüche 13, 38 oder 19, **dadurch gekennzeichnet, daß** man die Verbindungen der allgemeinen Formel I, worin der Rest $R^4$ für OH steht und die Reste $R^1$ bis $R^3$ die Bedeutung gemäß der allgemeinen Formel I haben in Gegenwart von Alkalihydroxiden und/oder Erdalkalihydroxiden und/oder metallorganischen Basen umsetzt.

**21.** Verfahren nach einem der Ansprüche 13 oder 18 bis 20, **dadurch gekennzeichnet, daß** man die Verbindungen der allgemeinen Formel I worin der Rest $R^4$ für OH steht und die Reste $R^1$ bis $R^3$ die Bedeutung gemäß der allgemeinen Formel I haben mit $C_{1-6}$-Alkylhalogeniden umsetzt.

**22.** Verfahren nach Anspruch 14, **dadurch gekennzeichnet, daß** man die Verbindungen der allgemeinen Formel I, worin der Rest $R^4$ für $OR^8$ steht und die Reste $R^1$ bis $R^3$ sowie $R^8$ die Bedeutung gemäß der allgemeinen Formel I haben, unter Schutzgasatmosphäre umsetzt.

**23.** Verfahren nach Anspruch 14 oder 22, **dadurch gekennzeichnet, daß** man die Verbindungen der allgemeinen Formel I, worin der Rest $R^4$ für $OR^8$ steht und die Reste $R^1$ bis $R^3$ sowie $R^8$ die Bedeutung gemäß der allgemeinen Formel I haben, in offenkettigen und/oder cyclischen Ethern umsetzt.

**24.** Verfahren nach einem der Ansprüche 14, 22 oder 23, **dadurch gekennzeichnet, daß** die eingesetzten cyclischen Carbonate 5 oder 6 Atome im Ring enthalten.

**25.** Arzneimittel enthaltend als pharmazeutischen Wirkstoff wenigstens ein substituiertes Pyrrolidin-2,3,4-trion-Derivat der allgemeinen Formel I gemäß Anspruch 1 und/oder eine entsprechende Base und/oder ein entsprechendes physiologisch verträgliches Salz und gegebenfalls weitere Wirkstoffe und/oder Hilfsstoffe.

**26.** Arzneimittel nach Anspruch 25 zur Behandlung/Bekämpfung bei/von Schmerzen, inflammatorischen und/oder allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen oder Epilepsie.

**27.** Arzneimittel nach Anspruch 25 zur Behandlung/Prophylaxe von/bei Schizophrenie, Morbus Alzheimer, Morbus Huntington, Morbus Parkinson, cerebralen Ischämien, cerebralen Infarkten, Psychosen bedingt durch erhöhten Aminosäurespiegel, Schlaganfällen, Hirnödemen, Hypoxie, Anoxie, AIDS-Demens, Encephalomyelitis, Tourette-Syndrom, perinataler Asphyxie oder zur Anxiolyse.

**28.** Verwendung wenigstens eines substituierten Pyrrolidin 2,3,4-trion-Derivates der allgemeinen Formel I gemäß Anspruch 1 und/oder einer entsprechenden Base und/oder eines entsprechenden physiologisch verträglichen Salzes

zur Herstellung eines Arzneimittels zur Bekämpfung von Schmerzen.

**29.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von inflammatorischen Reaktionen.

**30.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von allergischen Reaktionen.

**31.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Depressionen.

**32.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Drogen- und/oder Alkoholmißbrauch.

**33.** Verwendung wenigstens eines substituierten-Pyrrolidin-2,3,4-trion-Derivates der allgemeinen Formel I gemäß Anspruch 1 und/oder einer entsprechenden Base und/oder eines entsprechenden physiologisch verträglichen, Salzes zur Herstellung eines Arzneimittels zur Behandlung von Gastritis.

**34.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Diarrhoe.

**35.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Harninkontinenz.

**36.** Verwendung wenigstens eines substituierten Pyrrolidin-2,3;4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von cardiovaskulären Erkrankungen.

**37.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Atemwegserkrankungen.

**38.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Husten.

**39.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von seelischen Erkrankungen.

**40.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Epilepsie.

**41.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Schizophrenie.

**42.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Morbus Alzheimer.

**43.** , Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Morbus Huntington.

**44.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Morbus Parkinson.

**45.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von cerebralen Ischämien.

**46.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von cerebralen Infarkten.

**47.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Psychosen bedingt durch erhöhten Aminosäurespiegel.

**48.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Prophylaxe bei Schlaganfällen.

**49.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Hirnödemen.

**50.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Hypoxie.

**51.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Anoxie.

**52.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von AIDS-Demens.

**53.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von Encephalomyelitis.

**54.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung bei Tourette-Syndrom.

**55.** Verwendung wenigstens eines substituierten Pyrrolidin-2,3,4-trion-Derivates der allgemeinen Formel I gemäß Anspruch 1 und/oder einer entsprechenden Base und/oder eines entsprechenden physiologisch verträglichen Salzes zur Herstellung eines Arzneimittels zur Behandlung von perinataler Asphyxie.

**56.** Verwendung wenigstens eines substituierten Pyrrolidin-2,3,4-trion-3-oxim-Derivates der allgemeinen Formel I gemäß Anspruch 1 und/oder einer entsprechenden Base und/oder eines entsprechenden physiologisch verträglichen Salzes zur Herstellung eines Arzneimittels zur anxiolytischen Behandlung.

**Claims**

1. Substituted pyrrolidine-2,3,4-trione derivatives of the general formula I wherein

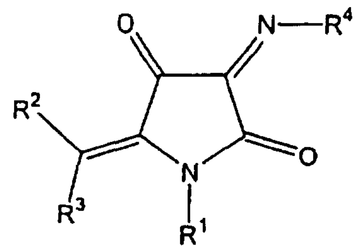

I

the radical $R^1$ represents H, $OR^8$, $COR^5$, $CSR^5$, $NR^6R^7$, $COOR^5$, $CONR^6R^7$, $CSNR^6R^7$, a $C_{1-10}$-alkyl or an unsubstituted phenyl radical.
the radicals $R^2$, $R^3$, which are identical or different, represent H, F, Cl, Br, $CF_3$, $OR^8$ $SR^8$, a $C_{1-10}$-alkyl, an aryl or a heteroaryl radical or represent an aryl radical bonded via a $C_{1-6}$-alkylene group,
the radical $R^4$ represents H, OH, $OR^8$, $SR^8$, $COR^5$, $COOR^5$, $COCOR^5$, $CONR^6R^7$, $CSNR^6R^7$ or a $C_{1-10}$-alkyl radical,
the radical $R^5$ represents H or a $C_{1-10}$-alkyl radical,
the radicals $R^6$, $R^7$, which are identical or different, represent H, $OR^8$, $COR^5$, $COOR^5$ or a $C_{1-10}$-alkyl, radical,
the radical $R^8$ represents a $C_{1-10}$-alkyl radical,
in the form of their racemates, enantiomers, diastereomers or a corresponding base or a corresponding physiologically tolerated salt.

2. Substituted pyrrolidine-2,3,4-trione derivatives according to claim 1, **characterized in that** the radical $R^1$ represents a $C_{1-6}$-alkyl radical and the other radicals $R^2$ to $R^8$ have the meaning according to the general formula I.

3. Substituted pyrrolidine-2,3,4-trione derivatives according to claim 1 or 2, **characterized in that** the radical $R^2$ and/or $R^3$ represents a $C_{1-6}$-alkyl radical and the other radicals $R^4$ to $R^8$ and where appropriate $R^2$ or $R^3$ have the meaning according to the general formula I.

4. Substituted pyrrolidine-2,3,4-trione derivatives according to claim 1 or 2, **characterized in that** the radical $R^2$ and/or $R^3$ represents an aryl radical bonded via a $C_{1-3}$-alkylene group and the other radicals $R^4$ to $R^8$ and where appropriate $R^2$ or $R^3$ have the meaning according to the general formula I.

5. Substituted pyrrolidine-2,3,4-trione derivatives according to one of claims 1 to 4, **characterized in that** the radical $R^4$ represents OH and the other radicals $R^5$ to $R^8$ have the meaning according to the general formula I.

6. Substituted pyrrolidine-2,3,4-trione derivatives according to one of claims 1 to 4, **characterized in that** the radical $R^4$ represents $OR^8$ and the other radicals $R^5$ to $R^8$ have the meaning according to the general formula I.

7. Substituted pyrrolidine-2,3,4-trione derivatives according to one of claims 1 to 4, **characterized in that** the radical $R^4$ represents a $C_{1-6}$-alkyl radical and the other radicals $R^5$ to $R^8$ have the meaning according to the general formula I.

8. Substituted pyrrolidine-2,3,4-trione derivatives according to one of claims 1 to 7, **characterized in that** the radical $R^5$ represents a $C_{1-6}$-alkyl radical and the other radicals $R^6$ to $R^8$ have the meaning according to the general formula I.

9. Substituted pyrrolidine-2,3,4-trione derivatives according to one of claims 1 to 8, **characterized in that** the radical $R^6$ and/or $R^7$ represents a $C_{1-6}$-alkyl radical and the radical $R^8$ and where appropriate $R^6$ or $R^7$ has the meaning according to the general formula I.

10. Substituted pyrrolidine-2,3,4-trione derivatives according to one of claims 1 to 9, **characterized in that** the radical $R^8$ represents a $C_{1-6}$-alkyl radical.

11. Substituted pyrrolidine-2,3,4-trione 3-oxime derivatives according to claim 1
5-(methoxyphenylmethylene)-pyrrolidine-2,3,4-trione 3-oxime,
5-(bromophenylmethylene)-pyrrolidine-2,3,4-trione 3-oxime.

12. Process for the preparation of substituted pyrrolidine-2,3,4-trione derivatives of the general formula I according to claim 1, **characterized in that** tetramic acids of the general formula II

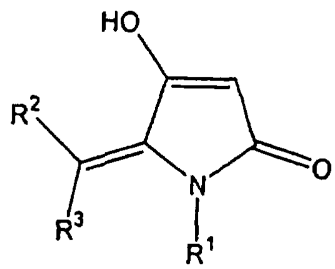

II

wherein the radicals $R^1$ to $R^3$ have the meaning according to the general formula I, are reacted with an aqueous solution of sodium nitrite in an ice-cooled solution, to give compounds of the general formula I wherein the $R^4$ represents OH and the radicals $R^1$ to $R^3$ have the meaning according to the general formula I, and these are purified and isolated.

13. Process for the preparation of substituted pyrrolidine-2,3,4-trione derivatives of the general formula I according to claim 1, **characterized in that** the compounds of the general formula I wherein the radical $R^4$ represents OH and the radicals $R^1$ to $R^3$ have the meaning according to the general formula I are reacted with $C_{1-10}$-alkyl halides in absolute solvents at low temperatures in the presence of strong bases to give compounds of the general formula I wherein the radical $R^4$ represents $OR^8$ and the radicals $R^1$ to $R^3$ and $R^8$ have the meaning according to the general formula I.

14. Process for the preparation of substituted pyrrolidine-2,3,4-trione derivatives of the general formula I according to claim 1, **characterized in that** the compounds of the general formula I wherein the radical $R^4$ represents $OR^8$ and the radicals $R^1$ to $R^3$ and $R^8$ have the meaning according to the general formula I are reacted with acid chlorides of the general formula $R^5$-(C=O)-Cl and/or acid bromides of the general formula $R^5$-(C=O)-Br or chloroformic acid esters of the general formula Cl-(C=O)-O-$R^5$ or fluoroformic acid esters of the general formula F-(C=O)-O-$R^5$ or with open-chain carbonates of the general formula $R^5$-O-(C=O)-O-$R^5$ or with correspondingly substituted cyclic carbonates, wherein in each case the radical $R^5$ has the meaning according to the general formula I, in an absolute solvent to give compounds of the general formula I wherein the radical $R^4$ represents $COR^5$ or $COOR^5$ and the radicals $R^1$ to $R^3$ and $R^5$ have the meaning according to the general formula I, and are purified and isolated by conventional processes.

15. Process for the preparation of substituted pyrrolidine-2,3,4-trione derivatives of the general formula I according to claim 1, **characterized in that** the compounds of the general formula I wherein the radical $R^4$ represents OH and the radicals $R^1$ to $R^3$ have the meaning according to the general formula I are reacted with aliphatic isocyanates or isothiocyanates at low temperatures in aprotic polar solvents to give compounds of the general formula I wherein $R^4$ represents $CONR^6R^7$ or $CSNR^6R^7$, the radical $R^6$ or $R^7$ denotes H and the radicals $R^1$ to $R^3$ and $R^6$ or $R^7$ have the meaning according to the general formula I, and are purified and isolated by conventional processes.

16. Process according to claim 12, **characterized in that** the tetramic acids of the general formula II are reacted with an aqueous solution of sodium nitrite in an ice-cooled solution of glacial acetic acid.

17. Process according to claim 12 or 16, **characterized in that** the compounds of the general formula I wherein the

radical $R^4$ represents OH and the radicals $R^1$ to $R^3$ have the meaning according to the general formula I are purified and isolated by recrystallization, preferably by recrystallization from ethanol.

**18.** Process according to claim 13, **characterized in that** the compounds of the general formula I wherein $R^4$ represents OH and the radicals $R^1$ to $R^3$ have the meaning according to the general formula I are reacted under an inert gas atmosphere.

**19.** Process according to claim 13 or 18, **characterized in that** the compounds of the general formula I wherein $R^4$ represents OH and the radicals $R^1$ to $R^3$ have the meaning according to the general formula I are reacted in open-chain and/or cyclic ethers.

**20.** Process according to one of claims 13, 18 or 19, **characterized in that** the compounds of the general formula I wherein the radical $R^4$ represents OH and the radicals $R^1$ to $R^3$ have the meaning according to the general formula I are reacted in the presence of alkali metal hydroxides and/or alkaline earth metal hydroxides and/or organometallic bases.

**21.** Process according to one of claims 13 or 18 to 20, **characterized in that** the compounds of the general formula I wherein the radical $R^4$ represents OH and the radicals $R^1$ to $R^3$ have the meaning according to the general formula I are reacted with $C_{1-6}$-alkyl halides.

**22.** Process according to claim 14, **characterized in that** the compounds of the general formula I wherein the radical $R^4$ represents $OR^8$ and the radicals $R^1$ to $R^3$ and $R^8$ have the meaning according to the general formula I are reacted under an inert gas atmosphere.

**23.** Process according to claim 14 or 22, **characterized in that** the compounds of the general formula I wherein the radical $R^4$ represents $OR^8$ and the radicals $R^1$ to $R^3$ and $R^8$ have the meaning according to the general formula I are reacted in open-chain and/or cyclic ethers.

**24.** Process according to one of claims 14, 22 or 23, **characterized in that** the cyclic carbonates employed contain 5 or 6 atoms in the ring.

**25.** Medicaments comprising, as the pharmaceutical active compound, at least substituted pyrrolidine-2,3,4-trione derivative of the general formula I according to claim 1 and/or a corresponding base and/or a corresponding physiologically tolerated salt, and optionally further active compounds and/or auxiliaries.

**26.** Medicaments according to claim 25 for treatment/control for/of pain, inflammatory and/or allergic reactions, depressions, drug and/or alcohol abuse, gastritis, diarrhoea, urinary incontinence, cardiovascular diseases, respiratory tract diseases, coughing, mental illnesses and/or epilepsy.

**27.** Medicaments according to claim 25 for treatment/prophylaxis of/for schizophrenia, Alzheimer's disease, Huntington's disease, Parkinson's disease, cerebral ischaemias, cerebral infarctions, psychoses caused by increased amino acid levels, apoplexies, cerebral oedemas, hypoxia, anoxia, AIDS dementia, encephalomyelitis, Tourette's syndrome, perinatal asphyxia or for anxiolysis.

**28.** Use of at least one substituted pyrrolidine-2,3,4-trione derivative of the general formula I according to claim 1 and/or a corresponding base and/or a corresponding physiologically tolerated salt for the preparation of a medicament for controlling pain.

**29.** Use of at least one substituted pyrrolidine-2,3,4-trione derivative of the general formula I according to claim 1 and/or a corresponding base and/or a corresponding physiologically tolerated salt for the preparation of a medicament for treatment of inflammatory reactions.

**30.** Use of at least one substituted pyrrolidine-2,3,4-trione derivative of the general formula I according to claim 1 and/or a corresponding base and/or a corresponding physiologically tolerated salt for the preparation of a medicament for treatment of allergic reactions

**31.** Use of at least one substituted pyrrolidine-2,3,4-trione derivative of the general formula I according to claim 1 and/or a corresponding base and/or a corresponding physiologically tolerated salt for the preparation of a medicament

for treatment of depressions.

**32.** Use of at least one substituted pyrrolidine-2,3,4-trione derivative of the general formula I according to claim 1 and/or a corresponding base and/or a corresponding physiologically tolerated salt for the preparation of a medicament for treatment of drug and/or alcohol abuse.

**33.** Use of at least one substituted pyrrolidine-2,3,4-trione derivative of the general formula I according to claim 1 and/or a corresponding base and/or a corresponding physiologically tolerated salt for the preparation of a medicament for treatment of gastritis.

**34.** Use of at least one substituted pyrrolidine-2,3,4-trione derivative of the general formula I according to claim 1 and/or a corresponding base and/or a corresponding physiologically tolerated salt for the preparation of a medicament for treatment of diarrhoea.

**35.** Use of at least one substituted pyrrolidine-2,3,4-trione derivative of the general formula I according to claim 1 and/or a corresponding base and/or a corresponding physiologically tolerated salt for the preparation of a medicament for treatment of urinary incontinence.

**36.** Use of at least one substituted pyrrolidine-2,3,4-trione derivative of the general formula I according to claim 1 and/or a corresponding base and/or a corresponding physiologically tolerated salt for the preparation of a medicament for treatment of cardiovascular diseases.

**37.** Use of at least one substituted pyrrolidine-2,3,4-trione derivative of the general formula I according to claim 1 and/or a corresponding base and/or a corresponding physiologically tolerated salt for the preparation of a medicament for treatment of respiratory tract diseases.

**38.** Use of at least one substituted pyrrolidine-2,3,4-trione derivative of the general formula I according to claim 1 and/or a corresponding base and/or a corresponding physiologically tolerated salt for the preparation of a medicament for treatment of coughing.

**39.** Use of at least one substituted pyrrolidine-2,3,4-trione derivative of the general formula I according to claim 1 and/or a corresponding base and/or a corresponding physiologically tolerated salt for the preparation of a medicament for treatment of mental illnesses.

**40.** Use of at least one substituted pyrrolidine-2,3,4-trione derivative of the general formula I according to claim 1 and/or a corresponding base and/or a corresponding physiologically tolerated salt for the preparation of a medicament for treatment of epilepsy.

**41.** Use of at least one substituted pyrrolidine-2,3,4-trione derivative of the general formula I according to claim 1 and/or a corresponding base and/or a corresponding physiologically tolerated salt for the preparation of a medicament for treatment of schizophrenia.

**42.** Use of at least one substituted pyrrolidine-2,3,4-trione derivative of the general formula I according to claim 1 and/or a corresponding base and/or a corresponding physiologically tolerated salt for the preparation of a medicament for treatment of Alzheimer's disease.

**43.** Use of at least one substituted pyrrolidine-2,3,4-trione derivative of the general formula I according to claim 1 and/or a corresponding base and/or a corresponding physiologically tolerated salt for the preparation of a medicament for treatment of Huntington's disease.

**44.** Use of at least one substituted pyrrolidine-2,3,4-trione derivative of the general formula I according to claim 1 and/or a corresponding base and/or a corresponding physiologically tolerated salt for the preparation of a medicament for treatment of Parkinson's disease.

**45.** Use of at least one substituted pyrrolidine-2,3,4-trione derivative of the general formula I according to claim 1 and/or a corresponding base and/or a corresponding physiologically tolerated salt for the preparation of a medicament for treatment of cerebral ischaemias.

46. Use of at least one substituted pyrrolidine-2,3,4-trione derivative of the general formula I according to claim 1 and/or a corresponding base and/or a corresponding physiologically tolerated salt for the preparation of a medicament for treatment of cerebral infarctions.

47. Use of at least one substituted pyrrolidine-2,3,4-trione derivative of the general formula I according to claim 1 and/or a corresponding base and/or a corresponding physiologically tolerated salt for the preparation of a medicament for treatment of psychoses caused by increased amino acid levels.

48. Use of at least one substituted pyrrolidine-2,3,4-trione derivative of the general formula I according to claim 1 and/or a corresponding base and/or a corresponding physiologically tolerated salt for the preparation of a medicament for prophylaxis of apoplexies.

49. Use of at least one substituted pyrrolidine-2,3,4-trione derivative of the general formula I according to claim 1 and/or a corresponding base and/or a corresponding physiologically tolerated salt for the preparation of a medicament for treatment of cerebral oedemas.

50. Use of at least one substituted pyrrolidine-2,3,4-trione derivative of the general formula I according to claim 1 and/or a corresponding base and/or a corresponding physiologically tolerated salt for the preparation of a medicament for treatment of hypoxia.

51. Use of at least one substituted pyrrolidine-2,3,4-trione derivative of the general formula I according to claim 1 and/or a corresponding base and/or a corresponding physiologically tolerated salt for the preparation of a medicament for treatment of anoxia.

52. Use of at least one substituted pyrrolidine-2,3,4-trione derivative of the general formula I according to claim 1 and/or a corresponding base and/or a corresponding physiologically tolerated salt for the preparation of a medicament for treatment of AIDS dementia.

53. Use of at least one substituted pyrrolidine-2,3,4-trione derivative of the general formula I according to claim 1 and/or a corresponding base and/or a corresponding physiologically tolerated salt for the preparation of a medicament for treatment of encephalomyelitis.

54. Use of at least one substituted pyrrolidine-2,3,4-trione derivative of the general formula I according to claim 1 and/or a corresponding base and/or a corresponding physiologically tolerated salt for the preparation of a medicament for treatment in cases of Tourette's syndrome.

55. Use of at least one substituted pyrrolidine-2,3,4-trione derivative of the general formula I according to claim 1 and/or a corresponding base and/or a corresponding physiologically tolerated salt for the preparation of a medicament for treatment of perinatal asphyxia.

56. Use of at least one substituted pyrrolidine-2,3,4-trione derivative of the general formula I according to claim 1 and/or a corresponding base and/or a corresponding physiologically tolerated salt for the preparation of a medicament for anxiolytic treatment.

## Revendications

1. Dérivés substitués de pyrrolidine-2,3,4-trione de formule générale I dans laquelle

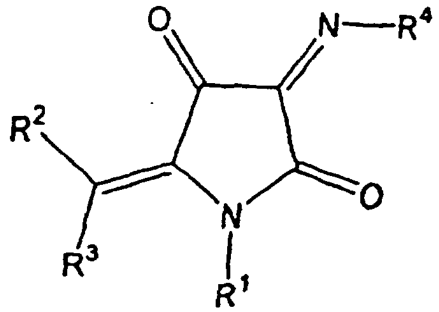

I

le reste $R^1$ représente H, $OR^8$, $COR^5$, $CSR^5$, $NR^6R^7$, $COOR^5$, $CONR^6R^7$, $CSNR^6R^7$, un reste alkyle en $C_1$ à $C_{10}$ ou un reste phényle non substitué,

les restes $R^2$, $R^3$, identiques ou différents, représentent H, F, Cl, Br, $CF_3$, $OR^8$, $SR^8$, un reste alkyle en $C_1$ à $C_{10}$, un reste aryle, un reste hétéroaryle ou un reste aryle lié par l'intermédiaire d'un groupe alkyle en $C_1$ à $C_6$,

le reste $R^4$ représente H, OH, $OR^8$, $SR^8$, $COR^5$, $COOR^5$, $COCOR^5$, $CONR^6R^7$, $CSNR^6R^7$ ou un reste alkyle en $C_1$ à $C_{10}$,

le reste $R^5$ représente H ou un reste alkyle en $C_1$ à $C_{10}$,

les restes $R^6$, $R^7$, identiques ou différents, représentent H, $OR^8$, $COR^5$, $COOR^5$, ou un reste alkyle en $C_1$ à $C_{10}$,

le reste $R^8$ représente un reste alkyle en $C_1$ à $C_{10}$,

sous forme de leurs racémates, de leurs énantiomères, de leurs diastéréoisomères ou d'une base correspondante ou d'un sel correspondant acceptable du point de vue physiologique.

2. Dérivés substitués de pyrrolidine-2,3,4-trione suivant la revendication 1, **caractérisés en ce que** le reste $R^1$ représente un reste alkyle en $C_1$ à $C_6$ et les autres restes $R^2$ à $R^8$ ont la définition selon la formule générale I.

3. Dérivés substitués de pyrrolidine-2,3,4-trione suivant la revendication 1 ou 2, **caractérisés en ce que** le reste $R^2$ et/ou le reste $R^3$ représentent un reste alkyle en $C_1$ à $C_6$ et les autres restes $R^4$ à $R^8$ de même que, éventuellement, $R^2$ ou $R^3$ ont la définition indiquée selon la formule générale I.

4. Dérivés substitués de pyrrolidine-2,3,4-trione suivant la revendication 1 ou 2, **caractérisés en ce que** le reste $R^2$ et/ou le reste $R^3$ représentent un reste aryle lié par l'intermédiaire d'un groupe alkylène en $C_1$ à $C_3$ et les autres restes $R^4$ à $R^8$ ainsi que, le cas échéant, $R^2$ ou $R^3$ ont la définition indiquée selon la formule générale I.

5. Dérivés substitués de pyrrolidine-2,3,4-trione suivant l'une des revendications 1 à 4, **caractérisés en ce que** le reste $R^4$ représente OH et les autres restes $R^5$ à $R^8$ ont la définition indiquée selon la formule générale I.

6. Dérivés substitués de pyrrolidine-2,3,4-trione suivant l'une des revendications 1 à 4, **caractérisés en ce que** le reste $R^4$ représente $OR^8$ et les autres restes $R^5$ à $R^8$ ont la définition indiquée selon la formule générale I.

7. Dérivés substitués de pyrrolidine-2,3,4-trione suivant l'une des revendication 1 à 4, **caractérisés en ce que** le reste $R^4$ représente un reste alkyle en $C_1$ à $C_6$ et les autres restes $R^5$ à $R^8$ ont la définition indiquée selon la formule générale I.

8. Dérivés substitués de pyrrolidine-2,3,4-trione suivant l'une des revendications 1 à 7, **caractérisés en ce que** le reste $R^5$ représente un reste alkyle en $C_1$ à $C_6$ et les autres restes $R^6$ à $R^8$ ont la définition indiquée selon la formule générale I.

9. Dérivés substitués de pyrrolidine-2,3,4-trione suivant l'une des revendications 1 à 8, **caractérisés en ce que** le reste $R^6$ et/ou le reste $R^7$ représentent un reste alkyle en $C_1$ à $C_6$ et le reste $R^8$ ainsi que, le cas échéant, le reste $R^6$ ou $R^7$ ont la définition indiquée selon la formule générale I.

**10.** Dérivés substitués de pyrrolidine-2,3,4-trione suivant l'une des revendications 1 à 9, **caractérisés en ce que** le reste $R^8$ représente un reste alkyle en $C_1$ à $C_6$.

**11.** A titre de dérivés substitués 3-oxime de pyrrolidine-2,3,4-trione suivant la revendication 1,
la 3-oxime de 5-(méthoxyphénylméthylène)-pyrrolidine-2,3,4-trione, et
la 3-oxime de 5-(bromophénylméthylène)-pyrrolidine-2,3,4-trione.

**12.** Procédé de production de dérivés substitués de pyrrolidine-2,3,4-trione de formule générale I suivant la revendication 1, **caractérisé en ce qu'**on fait réagir des acides tétramiques de formule générale II

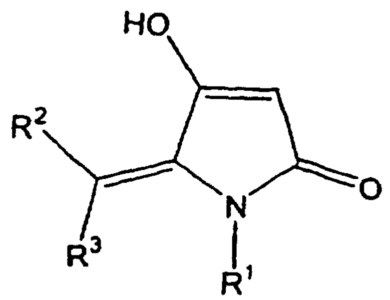

II

dans laquelle les restes $R^1$ à $R^3$ ont la définition indiquée selon la formule générale I, en solution refroidie à la glace, avec une solution aqueuse de nitrite de sodium pour obtenir des composés de formule générale I, dans laquelle le reste $R^4$ représente OH et les restes $R^1$ à $R^3$ ont la définition indiquée selon la formule générale I, et on purifie et isole ces composés.

**13.** Procédé de production de dérivés substitués de pyrrolidine-2,3,4-trione de formule générale I suivant la revendication 1, **caractérisé en ce qu'**on fait réagir les composés de formule générale I dans laquelle le reste $R^4$ représente OH et les restes $R^1$ à $R^3$ ont la définition indiquée selon la formule générale I dans des solvants absolus à de basses températures en présence de bases fortes, avec des halogénures d'alkyle en $C_1$ à $C_{10}$ pour obtenir des composés de formule générale I dans laquelle le reste $R^4$ représente $OR^8$ et les restes $R^1$ à $R^3$ ainsi que $R^8$ ont la définition indiquée selon la formule générale I.

**14.** Procédé de production de dérivés substitués de pyrrolidine-2,3,4-trione de formule générale I suivant la revendication 1, **caractérisé en ce qu'**on fait réagir les composés de formule générale I dans laquelle le reste $R^4$ représente $OR^8$ et les restes $R^1$ à $R^3$ ainsi que $R^8$ ont la définition indiquée selon la formule générale I, dans un solvant absolu avec des chlorures d'acides de formule générale $R^5$-(C=O)-Cl et/ou des bromures d'acides de formule générale $R^5$-(C=O)-Br ou des esters d'acide chloroformique de formule générale Cl-(C=O)-O-$R^5$ ou des esters d'acide fluoroformique de formule générale F-(C=O)-O-$R^5$ ou avec des carbonates à chaîne ouverte de formule générale $R^5$-O-(C=O)-O-$R^5$ ou avec des carbonates cycliques substitués de façon correspondante, formules dans chacune desquelles le reste $R^5$ a la définition indiquée selon la formule générale I, pour obtenir des composés de formule générale I dans laquelle le reste $R^4$ représente $COR^5$ ou $COOR^5$ et des restes $R^1$ à $R^3$ ainsi que $R^5$ ont la définition indiquée selon la formule générale I, et on purifie et isole ces composés selon des modes opératoires usuels.

**15.** Procédé de production de dérivés substitués de pyrrolidine-2,3,4-trione de formule générale I suivant la revendication 1, **caractérisé en ce qu'**on fait réagir les composés de formule générale I dans laquelle le reste $R^4$ représente OH et les restes $R^1$ à $R^3$ ont la définition indiquée selon la formule générale I, avec des isocyanates ou isocyanates aliphatiques à de basses températures dans des solvants aprotiques polaires pour obtenir des composés de formule générale I dans laquelle $R^4$ est un reste $CONR^6R^7$ ou $CSNR^6R^7$, le reste $R^6$ ou $R^7$ représente H et l'on purifie et isole ces composés selon des modes opératoires usuels.

**16.** Procédé suivant la revendication 12, **caractérisé en ce qu'**on fait réagir les acides tétramiques de formule générale II dans une solution d'acide acétique cristallisable refroidie à la glace, avec une solution aqueuse de nitrite de

sodium.

**17.** Procédé suivant la revendication 12 ou 16, **caractérisé en ce qu'**on purifie et isole les composés de formule générale I dans laquelle le reste $R^4$ représente OH et les restes $R^1$ à $R^3$ ont la définition indiquée selon la formule générale I, par recristallisation, avantageusement par recristallisation dans l'éthanol.

**18.** Procédé suivant la revendication 13, **caractérisé en ce qu'**on fait réagir les composés de formule générale I dans laquelle $R^4$ représente OH et les restes $R^1$ à $R^3$ ont la définition indiquée selon la formule générale I sous atmosphère de gaz protecteur.

**19.** Procédé suivant la revendication 13 ou 18, **caractérisé en ce qu'**on fait réagir les composés de formule générale I dans laquelle $R^4$ représente OH et les restes $R^1$ à $R^3$ ont la définition indiquée selon la formule générale I, dans des éthers à chaîne ouverte et/ou cycliques.

**20.** Procédé suivant l'une des revendications 13, 18 ou 19, **caractérisé en ce qu'**on fait réagir les composés de formule générale I dans laquelle le reste $R^4$ représente OH et les restes $R^1$ à $R^3$ ont la définition indiquée selon la formule générale I, en présence d'hydroxydes alcalins et/ou d'hydroxydes alcalinoterreux et/ou de bases organométalliques.

**21.** Procédé suivant l'une des revendications 13 ou 18 à 20, **caractérisé en ce qu'**on fait réagir les composés de formule générale I dans laquelle le reste $R^4$ représente OH et les restes $R^1$ à $R^3$ ont la définition indiquée selon la formule générale I, avec des halogénures d'alkyle en $C_1$ à $C_6$.

**22.** Procédé suivant la revendication 14, **caractérisé en ce qu'**on fait réagir les composés de formule générale I dans laquelle le reste $R^9$ représente $OR^8$ et les restes $R^1$ à $R^3$ ainsi que $R^8$ ont la définition indiquée selon la formule générale I, sous atmosphère de gaz protecteur.

**23.** Procédé suivant la revendication 14 ou 22, **caractérisé en ce qu'**on fait réagir les composés de formule générale I dans laquelle le reste $R^4$ représente $OR^8$ et les restes $R^1$ à $R^3$ ainsi que $R^8$ ont la définition indiquée selon la formule générale I, dans des éthers à chaîne ouverte et/ou cycliques.

**24.** Procédé selon l'une des revendications 14, 22 ou 23, **caractérisé en ce que** les carbonates cycliques utilisés contiennent 5 ou 6 atomes dans leur noyau.

**25.** Médicament contenant comme substance pharmaceutique active au moins un dérivé substitué de pyrrolidine-2,3,4-trione de formule générale I suivant la revendication 1 et/ou une base correspondante et/ou un sel correspondant acceptable du point de vue physiologique et, le cas échéant, d'autres substances actives et/ou substances auxiliaires.

**26.** Médicament suivant la revendication 25, destinée à traiter/combattre des douleurs, des réactions inflammatoires et/ou allergiques, des dépressions, l'abus de drogues et/ou d'alcool, la gastrite, la diarrhée, l'incontinence urinaire, des maladies cardiovasculaires, des maladies des voies respiratoires, la toux, des maladies psychiques ou l'épilepsie.

**27.** Médicament suivant la revendication 25, destiné au traitement/à la prophylaxie de la schizophrénie, de la maladie d'Alzheimer ou de la maladie d'Huntington, de la maladie de Parkinson, de l'ischémie cérébrale, de l'infarctus cérébral, de psychoses liées à un taux élevé d'aminoacides, d'apoplexie cérébrale, d'oedème du cerveau, de l'hypoxie, de l'anoxie, de la démence liée au SIDA, de l'encéphalomyélite, du syndrome de Tourette, de l'asphyxie périnatale ou destiné à l'anxiolyse.

**28.** Utilisation d'au moins un dérivé substitué de pyrrolidine-2,3,4-trione de formule générale I suivant la revendication 1 et/ou d'une base correspondante et/ou d'un sel correspondant acceptable du point de vue physiologique pour la préparation d'un médicament destiné à combattre des douleurs.

**29.** Utilisation d'au moins un dérivé substitué de pyrrolidine-2,3,4-trione de formule générale I suivant la revendication 1 et/ou d'une base correspondante et/ou d'un sel correspondant acceptable du point de vue physiologique pour la préparation d'un médicament destiné à combattre des réactions inflammatoires.

**30.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de réactions allergiques.

**31.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de dépressions.

**32.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de l'abus de drogues et/ou d'alcool.

**33.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de la gastrite.

**34.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de la diarrhée.

**35.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de l'incontinence urinaire.

**36.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de maladies cardiovasculaires.

**37.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de maladies des voies respiratoires.

**38.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de la toux.

**39.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de maladies psychiques.

**40.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de l'épilepsie.

**41.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de la schizophrénie.

**42.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de la maladie d'Alzheimer.

**43.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de la maladie de Huntington.

**44.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue

physiologique pour la préparation d'un médicament destiné au traitement de la maladie de Parkinson.

**45.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement d'ischémies cérébrales.

**46.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement d'infarctus cérébraux.

**47.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de psychoses dues à des taux élevés d'aminoacides.

**48.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné à la prophylaxie d'apoplexies cérébrales.

**49.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement d'oedèmes cérébraux.

**50.** Utilisation d'au moins un dérivé substitué de pyrrolidine-2,3,4-trione de formule générale I suivant la revendication 1 et/ou d'une base correspondante et/ou d'un sel correspondant acceptable du point de vue physiologique pour la préparation d'un médicament destiné au traitement de l'hypoxie.

**51.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de l'anoxie.

**52.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de la démence liée au SIDA.

**53.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de l'encéphalomyélite.

**54.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement dans le cas du syndrome de Tourette.

**55.** Utilisation d'au moins un dérivé substitué de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné au traitement de l'asphyxie périnatale.

**56.** Utilisation d'au moins un dérivé substitué 3-oxime de pyrrolidine-2,3,4-trione de formule générale I suivant la revendication 1 et/ou d'au moins une base correspondante et/ou d'au moins un sel correspondant acceptable du point de vue physiologique pour la préparation d'un médicament destiné à un traitement anxiolytique.